# EUROPEAN PATENT SPECIFICATION

(11) **EP 1 675 617 B1**
(45) Date of publication and mention of the grant of the patent: **26.07.2017**
(21) Application number: 04789401.9
(22) Date of filing: 30.09.2004
(51) Int. Cl.: A61K 35/74, A61P 13/12, A61P 1/00

(54) **COMPOSITIONS FOR AUGMENTING KIDNEY FUNCTION**
ZUSAMMENSETZUNGEN ZUR VERSTÄRKUNG DER NIERENFUNKTION
COMPOSITIONS VISANT A AUGMENTER LA FONCTION RENALE

(30) Priority: 30.09.2003 US 676622; 30.09.2003 US 676558; 20.10.2003 US 689359; 18.03.2004 US 803211
(43) Date of publication of application: 05.07.2006
(73) Proprietor: Kibow Biotech, Inc., Pennsylvania 19073 (US)
(72) Inventor: RANGANATHAN, Natarajan, Broomall, PA 19008 (US)
(74) Representative: O'Connell, Maura
(86) International application number: PCT/US2004/032250
(87) International publication number: WO 2005/032591

(56) References cited:
- EP-A- 0 994 183
- WO-A-02/091833
- US-A1- 2002 192 202
- US-A1- 2003 147 995
- FRIEDMAN E A ET AL: "GUT-BASED UREMIA THERAPY: SIMULTANEOUS REMOVAL OF UREA, CREATININE, AND URIC ACID" JOURNAL OF THE AMERICAN SOCIETY OF NEPHROLOGY, WILLIAMS AND WILKINS, BALTIMORE, MD, US, vol. 12, 1 January 2001 (2001-01-01), page 71A/72A, XP008051527 ISSN: 1046-6673
- DUNN STEPHEN R ET AL: "Effect of oral administration of freeze-dried Lactobacillus acidophilus on small bowel bacterial overgrowth in patients with end stage kidney disease: Reducing uremic toxins and improving nutrition" INTERNATIONAL DAIRY JOURNAL, vol. 8, no. 5-6, May 1998 (1998-05), pages 545-553, XP002497724 & MEETING ON FUNCTIONAL FOODS: DESIGNER FOODS FOR THE FUTURE; CORK, IRELAND; SEPTEMBER 30-OCTOBER 2, 1997 ISSN: 0958-6946
- DUNN STEPHEN R ET AL: "Oral lactobacillus acidophilus (LBA) reduces blood levels of dimethylamine (DMA), a uremic toxin, and nitrosodimethylamine (NDMA), a carcinogen in ESRD patients: Potential use for probiotics in ESRD" JOURNAL OF THE AMERICAN SOCIETY OF NEPHROLOGY, vol. 11, no. Program and Abstract Issue, September 2000 (2000-09), page 318A, XP009106280 & 33RD ANNUAL MEETING OF THE AMERICAN SOCIETY OF NEPHROLOGY AND THE 2000 RENAL WEEK; TORONTO, ONTARIO, CANADA; OCTOBER 10-16, 2000 ISSN: 1046-6673
- HIDA MIHO ET AL: "Inhibition of the accumulation of uremic toxins in the blood and their precursors in the feces after oral administration of Lebenin, a lactic acid bacteria preparation, to uremic patients undergoing hemodialysis" NEPHRON, vol. 74, no. 2, 1996, pages 349-355, XP009106278 ISSN: 0028-2766
- PRAKASH S ET AL: "MICROENCAPSULATED GENETICALLY ENGINEERED LIVE E. COLI DH5 CELLS ADMINISTERED ORALLY TO MAINTAIN NORMAL PLASMA UREA LEVEL IN UREMIC RATS" NATURE MEDICINE, NATURE PUBLISHING GROUP, NEW YORK, NY, US, vol. 2, no. 8, 1 August 1996 (1996-08-01), pages 883-887, XP001070902 ISSN: 1078-8956

## Description

### Background of the Invention

Kidney disease is ranked fourth among the major diseases in the United States afflicting over 20 million Americans. More than 90,000 patients die each year because of kidney diseases. In recent years the number of chronic kidney failure patients has increased about 11 percent annually. About 80,000 Americans on dialysis die of various complications each year and more than 27,000 are on waiting lists for kidney transplants each year with only about 11,000 of these patients receiving transplants. Further, nearly 350,000 Americans suffer from end stage renal disease (ESRD), which is the final stage in chronic renal failure.

In normal, healthy humans, metabolic waste nitrogen is primarily excreted via the kidneys as urea, uric acid creatinine, etc. in the urine. However, in individuals with kidney disease, as well as a number of other diseases such as inborn errors in urea cycle enzyme deficit, waste nitrogen accumulates in the body thereby manifesting toxic symptoms. Hyperammonium can lead to mental retardation and, in severe cases, coma.

Hemo- or peritoneal-dialysis and renal transplant are the only treatment modalities. However, the economic costs of these treatment modalities are extremely high. For example, in 1996 in the United States alone, the annual cost of end stage renal disease (ESRD) treatment was over 14 billion dollars. In developing and underdeveloped countries with low health care budgets, ESRD patients are deprived access to such treatments due to their high costs. Accordingly, there is a need for alternative modalities of treatment for uremia.

A number of treatment attempts have been based on the use of the bowel as a substitute for kidney function. During a normal digestive process the gastrointestinal tract delivers nutrients and water to the bloodstream and eliminates some waste products and undigested materials through the bowel. The intestinal wall regulates absorption of nutrients, electrolytes, water and certain digestive aiding substances such as bile acids. The intestinal wall also acts as a semi-permeable membrane allowing small molecules to pass from the intestinal tract into the bloodstream and preventing larger molecules from entering the circulation.

Nitrogenous wastes such as urea, uric acid, creatinine and uric acid, along with several other small and medium molecular weight compounds, flow into the small intestine and equilibrate across the small intestine epithelium. Studies of intestinal dialysis have shown a daily flow of 71 grams of urea, 2.9 grams of creatinine, 2.5 grams of uric acid and 2.0 grams of phosphate into the intestinal fluid (Sparks (1975) Kidney Int. Suppl. 7(Suppl 3):373-376). Accordingly, various invasive and noninvasive attempts including external gut fistula, intestinal dialysis, induced diarrhea, and administration of oral sorbents and/or encapsulated urease enzyme have been made to extract uremic waste from the gastrointestinal tract (Twiss and Kolff (1951) JAMA 146:1019-1022; Clark et al. (1962) Trans. Am. Soc. Artif. Intrn. Organs 8:246-251; Pateras et al. (1965) Trans. Am. Soc. Artif. Intrn. Organs 11:292-295; Shimizu et al. (1955) Chemical Abstracts 103:129004; Kjellstrand et al. (1981) Trans. Am. Soc. Artif. Intern. Organs 27:24-29; Kolff (1976) Kidney Int. 10:S211-S214).

In addition, genetically engineered *E. herbicola* cells have been encapsulated and demonstrated to convert ammonia into usable amino acids for the cells before being eliminated via the bowel. Microencapsulated genetically engineered *E. coli* DH5 cells have also been shown to be effective in removal of urea and ammonia in an *in vitro* system and in a uremic rat animal model (Prakash and Chang (1995) Biotech. Bioengin. 46:621-26; Prakash and Chang (1996) Nature Med. 2:883-887).

The human gastrointestinal tract harbors a complex microbial ecosystem containing a large number and variety of bacteria. The resident bacterial population in the human gastrointestinal tract has a major impact on gastrointestinal function and thereby on human health and well-being. Among these, some bacteria are opportunistic or considered to be detrimental and cause adverse conditions such as diarrhea, infections, gastroenteritis and endotoxaemia, while some bacteria species are considered as "probiotic", in that they perform beneficial functions for the human organism (Holzapfel, et al. (1998) Int. J. Food Microbiol. 41(2):85-101).

Among the probiotic bacteria, *Bifidobacteria* species are the most prominent. *Bifidobacteria* species, when in live and viable form, stimulate the immune system and exert a competitive exclusion of pathogenic and putrefactive bacteria, reduce the amounts of ammonia and cholesterol in the blood, and promote absorption of minerals. In addition, *Bifidobacteria* have been suggested to exert a preventive action against colon cancer, by reducing the activity of some enzymes that convert procarcinogen substances into carcinogen substances (von Wright, et al. (1999) Eur. J. Gastroenterol. Hepatol. 11(11):1195-1198).

The lactic bacteria such as *Lactobacillus bulgaricus, Lactobacillus acidophilus, Lactobacillus casei, Lactobacillus plantarum* and *Streptococcus faecium. Streptococcus thermophilus* are also probiotic. These bacteria produce antagonist effects against pathogenic microorganisms, stimulate the immune system, improve lactose digestion, perform a lipolytic activity making fats more digestible, reduce plasmatic values of cholesterol, protect the intestinal mucosa ensuring an even assimilation of the nutritive substances, produce polysaccharides that are active on some tumors, and reduce viability of some enzyme-producing microorganisms catalyzing conversion of procarcinogen substances into carcinogenic substances.

It is believed that the probiotic bacteria exert their effects in a synergistic manner to curtail and retard the growth of pathogenic/detrimental bacteria of the gut (Marteau, et al. (2001) Am. J. Clin. Nutr. 73(2 Suppl):430S-436S; Cummings, et al. (2001) Am. J. Clin. Nutr. 73(2 Suppl):415S-420S).

The intestinal bacteria flora can be reduced, become unbalanced or be eliminated in patients undergoing antibiotic treatment and other therapies, and in individuals suffering from inflammatory intestinal diseases, kidney disease and liver disease. In addition, it has been shown that during normal aging the *Bifidobacteria* population is reduced while the concentration of pathogenic and putrefactive bacteria concomitantly increases (Orrhage, et al. (2000) Drugs Exp. Clin. Res. 26(3):95-111).

It is also known that beneficial effects of microbes such as the *Bifidobacterium* species are in part due to their ability to ferment nondigestible sugars, known as prebiotics, present in the colon. A prebiotic is a non-digestible food ingredient that beneficially affects the host by selectively stimulating the growth and/or the activity of one or a limited number of bacteria in the colon. Prebiotics are typically thought of as carbohydrates of relatively short chain length. Prebiotics are like other carbohydrates that reach the cecum, such as nonstarch polysaccharides, sugar alcohols, and resistant starch, in being substrates for fermentation. They are, however, distinctive in their selective effect on the microflora. To be effective, prebiotics they must reach the cecum (Bezkorovainy (2001) Am. J. Clin. Nutr. 73 (2 Suppl):399S-405S).

U.S. Patent 5,733,568 teaches the use of microencapsulated *Lactobacillus* bacteria for treatment of antibiotic-associated or other acute and chronic diarrhea as well as for skin and vaginal yeast infections. The microencapsulation is said to prevent inactivation of the bacillus and to deliver it to the intestine as well as to avoid lactose intolerance seen in said diarrheas.

U.S. Patent 5,032,399 teaches the use of species of *Lactobacillus acidophilus* to adhere to intestinal mucosa and thereby reduce gastrointestinal side effects of antibiotic therapy that reduces beneficial bacteria population.

U.S. Patent 5,531,988 teaches, in addition to beneficial bacteria, use of immunoglobulin in the composition as a dietary supplement.

U.S. Patent 5,840,318 also teaches a beneficial bacterial composition that can modulate the immune system of animals.

EP 0994183 discloses a composition comprising an untreated or treated culture of a mixed microorganism comprising at least 3 lactic acid bacteria.

Use of probiotics such as *Lactobacillus acidophilus* has been suggested to curtail the bacterial overgrowth and the accumulation of uremic toxins and carcinogenic compounds. Unabsorbable carbohydrate in the diet of uremic patients has also been shown to increase fecal nitrogen. Use of lactulose and dietary fiber has also been shown to reduce plasma urea 11 to 27% and increase fecal nitrogen excretion to 39 to 62% (Lange (1997) Nature Med. 3:2-3).

One of the major deficits of these prior art approaches, however, is that they tend to address individual uremic solutes or toxins. However, proper clinical management of renal, hepatic and gastrointestinal diseases or disorders actually requires alleviation of multiple symptoms. The present invention provides compositions for use in augmenting kidney function.

### Summary of the Invention

The present invention relates to a composition according to claim 1 for use in augmenting kidney function in a human or animal subject. The composition contains living probiotic bacteria which reduce creatinine and BUN levels in the subject. In other disclosures, the composition further contains at least one carbohydrate, at least one fat ingredient, at least one protein ingredient, at least one vitamin component, at least one mineral component, or at least one prebiotic. In another disclosure, the composition provides about 5 billion to 20 billion colony forming units of the probiotic bacterium.

The present invention further relates to a composition for use for augmenting kidney function using a probiotic composition so that build up of toxins and metabolic wastes, and overgrowth of undesirable bacteria is reduced in the subject thereby augmenting kidney function. In particular embodiments, the probiotic composition for use alleviates the symptoms of uremia; the increased creatinine or BUN levels in subjects undergoing cancer, HIV or AIDS chemotherapy treatment; the increased creatinine or BUN levels in subjects with metabolic syndrome; or the symptoms of increased creatinine or BUN levels in subjects consuming high protein and low carbohydrate diets.

The invention is set forth in the accompanying claims.

Methods for restoring and maintaining gastrointestinal health and delaying or eliminating the progression of a metabolic disease by administering to a subject a probiotic composition are also disclosed.

### Brief Description of the Drawings

Figure 1 shows the urea hydrolysis by *S. thermophilus* strains at pH 5.5 (Figure 1A), pH 6.3 (Figure 1B), and pH 7.5 (Figure 1C). AIF, 100 mg/dL urea with 100 µM NiCl₂. Data is presented as mean ± SEM, n= 3-9.
Figure 2 shows urea hydrolysis by *S. thermophilus* strains KB19 (Figure 2A), KB4 (Figure 2B), and KB25 (Figure 2C) at concentrations of urea characteristic of uremic blood levels. AIF, pH 6.3, with 100 µM NiCl₂. Data is presented as mean ± SEM, n= 3-5.
Figure 3 shows ureolysis and urea-nitrogen concentration degradation rates for different bacterial strains. Diamonds, control; circles, *Bacillus pasteurii* ATCC 6453 triangle, *Streptococcus thermophilus* KB19; squares, *E. coli* strain DH5α transformed with a multi-copy plasmid bearing the *Klebsiella aerogenes* urease operon.

### Detailed Description of the Invention

In kidney failure there is a decrease in the glomerular filtration rate and the kidneys are unable to maintain homeostasis of the blood. Homeostatic balance of water, sodium, potassium, calcium and other salts is no longer possible and nitrogenous wastes are not excreted. Retention of water causes edema and as the concentration of hydrogen ions increases, acidosis develops. Nitrogenous wastes accumulate and a condition referred to as uremia develops in the blood and tissue. Uremic toxins can be defined as solutes that are normally excreted by healthy kidneys, accumulate progressively during the development of renal failure so that their concentration increases, and inhibit various physiologic and biochemical functions; as a whole, they contribute to a complex set of clinical symptoms that comprise the uremic syndrome. Examples of uremic toxins include, but are not limited to, ammonia, urea, creatinine, phenols, indoles, and middle molecular weight molecules. Urea levels are expressed as blood urea nitrogen readings. More specifically, in uremia, the concentration of serum creatinine, blood urea nitrogen (BUN), uric acid, and guanidino compounds such as N-methyl guanidine (NMG) and guanidino succinic acid (GSA) are significantly altered with accompanying abnormalities in acid-base equilibrium, electrolytes and water retention. Urea is a chemical that the liver makes from ammonia. Ammonia is absorbed in the gut from the breakdown of dietary protein. After production in the liver, urea is excreted by the kidneys. Urea increases in other disease states as well including dehydration and urethral obstruction. The creatinine is a breakdown product of muscle and is excreted by the kidney at a constant rate. Creatinine serves as an important marker for kidney function. In renal failure the phosphorous levels in a subject may increase and lead to mineralization of various sites in the body. In addition there are several known and unknown substances of low and middle molecular weight which have been identified as uremic toxins which also accumulate. If untreated, acidosis and uremia can cause coma and eventually death.

The introduction of renal dialysis has contributed to rapid progress in the clinical treatment of renal failure and elucidation of uremia. When a patient has mild kidney failure where the serum creatinine level exceeds the normal range of 1.2 mg/dL or less, the patient does not require renal replacement therapy such as dialysis or renal transplant. However, in general, when the serum creatinine level rises to 13.6±4.6 mg/dL, the patient needs routine dialysis or a kidney transplant to survive.

Dialysis can serve as a lifetime therapy for ESRD patients. Phosphate binders such as RENAGEL® (Geltex/Genzyme, Boston, Massachusetts), calcium acetate, calcium carbonate or aluminum hydroxide are generally prescribed for uremic patients receiving dialysis to reduce elevated phosphate levels. In general, however, dialysis is very expensive, inconvenient, time consuming and may occasionally produce one or more side effects. With a successful kidney transplant, a patient can live a more normal life with less long-term expense. However, there are also high costs associated with transplant surgery, the recovery period and the continuous need for anti-rejection medications. Further, there are often times a shortage of suitable donors. Accordingly, there is a need for alternative strategies.

The present invention provides a composition for use in augmenting kidney function, particularly in subjects with renal insufficiency, comprising living probiotic bacteria wherein said probiotic bacteria reduce creatinine and BUN levels in the subject. The composition may comprise an enteric coating so that the composition reaches the intestines of the subject when ingested orally.

The present invention provides a formulation of commensal and food grade bacteria known as probiotic bacteria, that when ingested become intestinal or gut flora and are capable of metabolizing urea and ammonia, preferably to amino acids which can be used by the bacteria or the patient. Instillation of such probiotic bacteria permits a reduction in frequency and even elimination of the need for dialysis. Probiotic bacteria of the present invention are living microorganisms that are naturally present in the gastrointestinal tract of humans and animals. They are beneficial bacteria that enhance the body's defenses against a number of health conditions. A probiotic bacterium that is disclosed includes: *Lactobacillus* sp. such as *L. acidophilus, L. bulgaricus, L. casei, L. rhamnosus, L. fermentum, L. salivaroes, L. brevis, L. plantarum, L. ruteri, or L. sporogenes; Streptococcus* sp. such as *S. thermophilus; Bacillus* sp. such as *B. pasteurii;* and *Bifidobacterium* sp. such as *B. adolescentis, B. infantis, B. longum, B. thermophilus,* or *B. bifidum.* In particular disclosures, the probiotic bacterium comprises one or more of the following: *L. bulgaricus, S. thermophilus, L. acidophilus, L. sporogenes* or *B. bifidum.* In other disclosures, the probiotic bacterium exhibits high urease activity (*e.g., B. pasteurii* or *S. thermophilus*)*.*

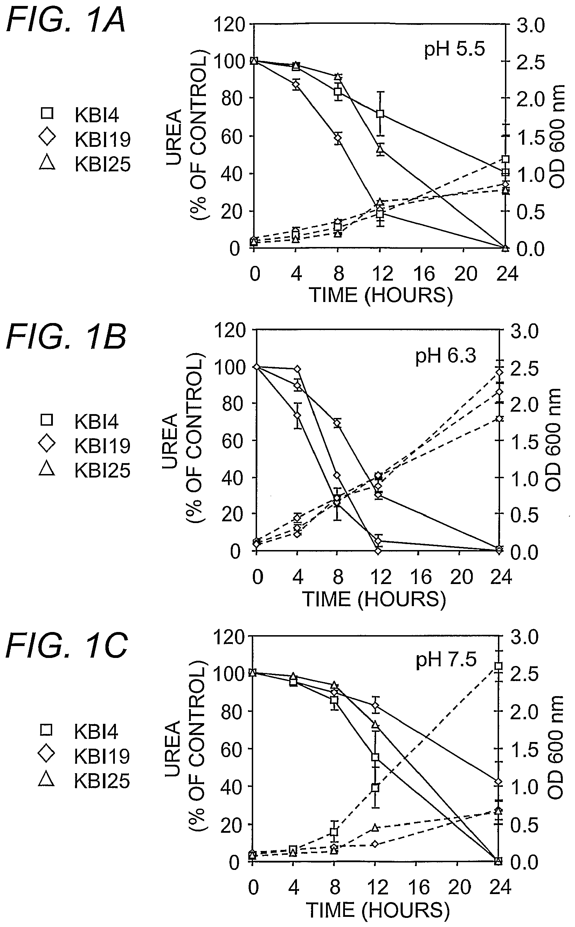

In particular embodiments, the probiotic bacterium may function to restore normal balance between beneficial bacteria and detrimental bacteria, and to remove excess urea-waste product of normal protein metabolism thereby reducing the burden on ailing kidney. It is desirable that the *S*. *thermophilus* strain used is K9, K19, or K25. These probiotic, urealytic isolates were isolated from different food sources and characterized *in vitro* by assessing their ability to catabolize urea while proliferating in simulated gastric juice (Table 1) and simulated intestinal fluid (Figures 1A-1C).

**TABLE 1**

| Strain | pH | Survival (cfu/mL) | | | |
|---|---|---|---|---|---|
| | | 0 Hours | 1 Hour | 2 Hours | 3 Hours |
| KB19 | 1.4 | 10¹⁰ | 0 | 0 | 0 |
| | 2.0 | 10¹⁰ | 0 | 0 | 0 |
| | 2.4 | 10¹⁰ | 10⁴ | 10⁴ | 10⁴ |
| | 3.0 | 10¹⁰ | 10⁸ | 10⁸ | 10⁶ |
| KB4 | 1.4 | 10¹⁰ | 10³ | 0 | 0 |
| | 2.0 | 10¹⁰ | 10³ | 0 | 0 |
| | 2.4 | 10¹⁰ | 10⁵ | 10⁴ | 10⁴ |
| | 3.0 | 10¹⁰ | 10¹⁰ | 10¹⁰ | 10⁹ |
| KB25 | 1.4 | 10¹⁰ | 0 | 0 | 0 |
| | 2.0 | 10¹⁰ | 0 | 0 | 0 |
| | 2.4 | 10¹⁰ | ND | ND | 10⁶ |
| | 3.0 | 10¹⁰ | ND | ND | 10⁷ |

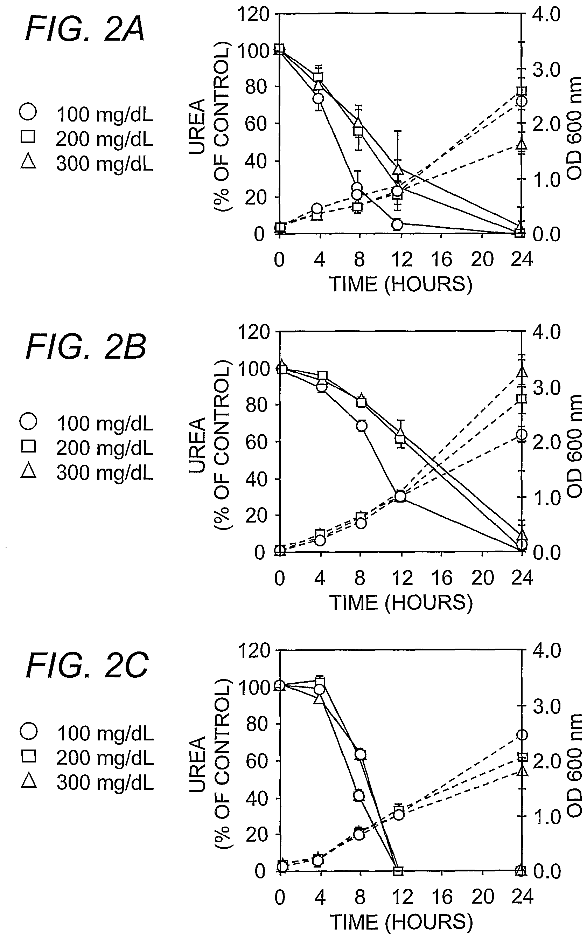

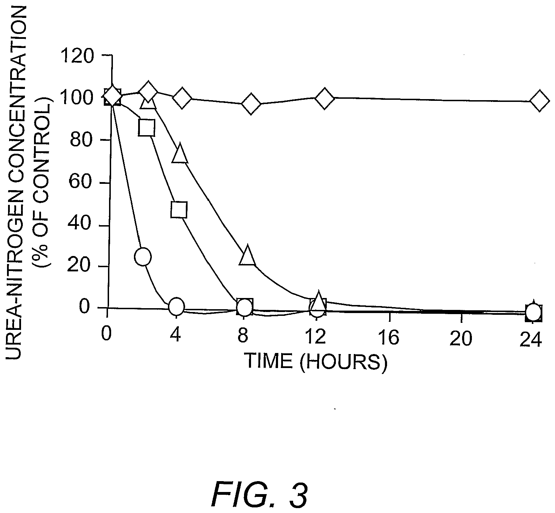

All three strains: proliferated in the fed state simulated artificial intestinal fluid (AIF) in the pH range from 5.5 to 7.5, characteristic of the colon environment; used urea as a sole nitrogen source; and catabolized urea in the presence of other nitrogen sources. Urea hydrolysis was growth- and pH-dependent. Figures 2A-2C show that KB19, KB4, and KB25, respectively, are efficient in hydrolyzing urea in blood. Under all the conditions tested, the rate of urea hydrolysis was strain dependent permitting selection of the best candidate for uremic applications. One selected isolate, *S. thermophilus* KB19, reduced urea concentrations from 300 mg/dL, to 20 mg/dL within 24 hours at pH 6.3 when inoculated at initial density of 10⁹ cfu/mL KB19 survived 3 hours in acidic pH 3.0 with only two logs loss in cfu and was able to pass through bile. Further, it was found that ureolysis by KB19 was comparable to other urea utilizing bacteria such as *B. pasteurii* and genetically engineered urealytic *E. coli* (Figure 3) when assessed in AIF. AIF was prepared according to U.S. pharmacopocia with modifications (addition of 1% dextrose, 100 µM NiCl₂, 10% MRS broth and 100 mg/dL urea and, for growth of the plasmid bearing *E.coli,* 0.01% ampicillin). Bacterial strains were inoculated into modified AIF at the initial density of 10⁹ cfu/mL for *S. thermophilus* and *B. pasteurii* and 10⁸ cfu/mL for *E.coli* and incubated at 37°C. Aliquots were taken at 0, 2, 4, 8, 12 and 24 hours and urea nitrogen and optical density (OD) data were recorded. Within 24 hours all strains removed 100% of urea from the system. Therefore, *S*. *thermophilus* KB19 is advantageously used in a composition of the present invention to remove urea. In addition, strain KB19 did not exhibit any resistance to commonly used antibiotics.

It has been found that probiotic bacteria are effective in ranges of from 10⁹ to 10¹¹ cfu (colony forming units). For instance, *Lactobacillus acidophilus* is of particular useful between 10⁹ to 10¹⁰ cfu, whereas *B. longum* is desirably used between 10⁹ to 10¹⁰ cfu, and *S. thermophilus* between 10¹⁰ to 10¹¹ cfu. See Table 3 of Example 1.

To illustrate the effectiveness of using a probiotic bacterium to reduce nitrogenous waste build up due to renal failure, probiotic compositions were fed to uremic rats. Sprague-Daly rats weighing 281.20±41.6 gm were subjected to 5/6th nephrectomy after measurement of baseline weight, BUN, serum creatinine, urine volume and fecal flora composition. The study group consisted of 36 nephrectomized rats (18 male and 18 female) with sufficient renal impairment (serum creatinine=1.0±0.4) and 6 controls. The rats were distributed into 6 matched groups (GP), ANOVA showed no significant difference between groups (p=0.516) at baseline. After a two-week post surgery stabilization, the cohorts of six rats were fed standard rat chow plus one of the following regimens: 1) placebo; 2) *B. pasteurii;* 3) *L. sporogenes;* 4) *L. acidophilus, L. bulgaricus, B. bifidus, S. thermophilus, L. casei,* and *L. reuteri;* 5) *L. acidophilus, L. bulgaricus, B. bifidus, S. thermophilus;* and 6) *S. boulardii.* A control group of non-nephrectomized rats (n=7, serum creatinine=0.2±0.1) received the same food without any supplement. All of the control rats survived and had serum creatinine levels at the end of the study of 0.5±0.1. For the experimental animals, blood, urine, and fecal measurements were obtained every 30 days for a total of 120 days. Days of survival was the primary endpoint variable (Table 2).

**TABLE 2**

| GP | Organism | ALIV E | DEA D | %SURVIV E | Mean Days±SD | Median |
|---|---|---|---|---|---|---|
| 6 | *S*. *boulardi* | 2 | 4 | 33.3 | 111±44 | 113 |
| 1 | Placebo | 2 | 4 | 33.3 | 116±39 | 122 |
| 5 | H1001 | 2 | 4 | 33.3 | 116±36 | 110 |
| 4 | SF101 | 3 | 3 | 50 | 126±33 | 132 |
| 2 | *B*. *pasteruii* | 4 | 2 | 66.7 | 148±14 | 156 |
| 3 | *L*. *sporogenes* | 5 | 1 | 83.3 | 149±16 | 156 |

As shown in Table 2, diets of *B. pasteurii* and L. *sporogenes* were more effective than the other regimes (p<0.05) at increasing survival in otherwise untreated uremic rats.

The nephrectomized rats fed *B. pasteurii* and L. *sporogenes* had lower BUN levels (62.0±21 mg/dL and 63.0±26 mg/dL, respectively) compared with placebo (99.0±46 mg/dL) a reduction of (38% and 37%, respectively). Serum creatinine levels were similarly reduced in rats fed with *B. pasteurii* and *L. sporogenes* (0.9±0.25 mg/dL and 0.9±0.2 mg/dL, respectively) compared to placebo (1.5±0.56 mg/dL) a reduction of 40% in both groups. The other regimens were less effective at reducing BUN or serum creatinine levels compared to placebo. Feeding increased the fecal count for the appropriate group of bacteria in all groups at eight weeks. These results indicate that *B. pasteurii* and *L*. *sporogenes* administered orally as dietary supplements metabolize urea and creatinine *in vivo* in subjects.

Compositions for use *in vivo* are advantageously enterically coated and/or microencapsulated, *i.e.,* in a gel cap or other desired form. Enteric coating of the composition is specifically designed to deliver the composition of the present invention at the ileal and colonic regions of the bowel where maximal resorption of uremic solutes and other molecules are found to occur. This is desirably achieved via a coating material that disintegrates and dissolves at a pH of 6.6 to 7.5 or higher. Examples of enteric coatings with these characteristics include, but are not limited to, Zein, polyglycolactic acid, polylactic acid, polylactide-co-glycolide and similar coating materials. In one embodiment, the coating is a double-layered gelatin coating.

Compositions can further contain a phosphate binding agent such as aluminum hydroxide gel, calcium carbonate or calcium acetate, magnesium hydroxide gel and/or a water binding agent such as psyllium fibers, naturally occurring gums such as locust bean gum, guar gum or modified starches.

Compositions can further contain at least one carbohydrate, at least one fat, at least one protein, and/or at least one prebiotic bacterium. While such additional additives can be combined with the one or more probiotic bacteria and administered in the form of a capsule, gel cap, or pill, it may be desirable to formulate the composition into a nutritional food or nutritional food product.

Probiotics are also contemplated as suitable ingredients for use in the compositions of the present invention. Prebiotic as used herein is meant to mean any non-living, non-digestible food ingredient that beneficially affects the host by selectively stimulating the growth and/or activity of one or a limited number of bacteria, including probiotic bacteria, in the colon, with the effect of improving the host's health. Suitable prebiotics include, but are not limited to, oligosaccharides such as fructo-oligosaccharides (such as, but not limited to, inulin), galacto-oligosaccharides, soy-oligosaccharides, xylo-oligosaccharides, isomalto-oligosaccharides, Jerusalem artichoke flour, rolled oats, banana fiber, pectins, pectic polysaccharides, mannans, guar gum, locust bean gum, konjac, and xanthan gum, pentosans, beta-glucans, arabinans or galactans, larch arabinogalactans, and mixtures thereof. A nutritional food or food product may contain from about 2.0%-6.0% prebiotic component(s). The prebiotics may be from about 3.0%-5.0%. Alternatively, the prebiotic may be about 4.0%-5.0%. All weights are based upon the total weight of the nutritional food or food product taken as 100% by weight.

Given the beneficial effects of the compositions of the present invention on the health and well-being of subjects, in particular subjects with renal insufficiency, the present invention relates to the inventive compositions for use in augmenting kidney function. While the compositions of the present invention are particularly useful to subjects with renal insufficiency, it is contemplated that healthy individuals will also benefit from the compositions of the present invention. Thus, in accordance with the methods of the present invention, a subject is intended to include healthy and ailing humans and animals, including pigs, horses, cats and dogs.

The compositions of the present invention are administered to a subject in the various forms disclosed herein in an amount which effectively decreases or inhibits the build up of toxins, decreases the build up of wastes and/or decreases the overgrowth of undesirable bacteria. Such an amount is readily determined without undue experimentation by those skilled in art. For example, it is desirable that the compositions of the present invention be administered to the subject on a routine basis such as one or more times daily over a period of time. Reduction of toxins and nitrogenous wastes is indicated via blood, urine or fecal sample testing wherein a stabilization or reduction in BUN levels or serum creatinine levels of the blood, urine or fecal samples as compared to initial or control levels indicates effective treatment.

In one embodiment of the present invention, augmentation of kidney function in a subject with uremia can result in the alleviation of symptoms of uremia. By alleviation of symptoms of uremia, it is meant that the composition removes sufficient levels of uremic toxins such that a patient suffering from uremia either does not require dialysis, requires dialysis less frequently or for shorter durations, or does not require initiation of dialysis as soon as would be needed without treatment. Compositions of the present invention can also be administered to a subject in need thereof to treat not only renal insufficiency and inborn error of urea metabolism, but also liver insufficiency and gastrointestinal disorders and diseases.

Toxins and waste products tend to accumulate in the gastrointestinal tract in any condition which disrupts the kidneys ability to excrete the build up of nitrogenous waste products in the blood, thereby resulting in the diffusion of the nitrogenous waste products from the circulating blood into the bowel. Conditions that affect nitrogen metabolism include, but are not limited to, high protein consumption, chemotherapy, metabolic diseases, defects in protein metabolism, and nucleic acid metabolism. Diabetes, kidney failure, and liver disease, as well as other conditions (e.g., chemotherapy for cancer, HIV, and AIDS; or a diet rich in protein and low in carbohydrates) can result in the build up of toxic nitrogenous compounds such as creatinine and urea in the blood. Accordingly, the compositions of the present invention are particularly useful in alleviating the increased creatinine or BUN levels in subjects undergoing cancer, HIV, or AIDS chemotherapy treatment, subjects with a metabolic syndrome, or individuals consuming a high protein/low carbohydrate diet.

Toxic nitrogenous waste products can also accumulate in the intestinal tract of a subject when the normal balance of intestinal microbes is disrupted. A healthy gastrointestinal tract with adequate mucus production and appropriate bacterial colonization prevents the overgrowth of pathogenic bacteria, modulates disease processes, and prevents widespread inflammatory disorders. A complex bacterial structure and diverse pecking order exist among the microorganisms that inhabit the gastrointestinal tract. To maintain and promote a healthy gastrointestinal tract it is important to effectively encourage the growth of beneficial bacteria while minimizing the proliferation and/or overgrowth of the detrimental bacteria. Therefore, the probiotic compositions provided herein will benefit the overall general condition of the gastrointestinal tract by providing probiotic bacteria to restore or maintain gastrointestinal health. This is especially beneficial to individuals with a propensity to gastrointestinal complaints. Exemplary gastrointestinal complaints include, but are not limited to, inflammatory conditions, such as, but not limited to, irritable bowel syndrome, inflammatory bowel disease, colitis, Crohn's disease; and diarrhea.

The precise amount of the compositions of the present invention ingested by a subject may be decided according to the judgment of a practitioner or dietician and each subject's circumstances.

The present invention is further illustrated by the following non-limiting examples.

### Example 1: BUN/Creatinine Decrease

Different formulations of probiotic compositions were tested on 5/6th nephrectomized mini pigs. From a total of 20 pigs obtained over a six month period, three died post-surgery (one of the three had to be euthanized due to extreme sickness) and two more were euthanized due to acute illness, possibly due to infection.

The mini pigs that were fed formulation 1 were subsequently separated and used again for feeding formulations 2A and 2B. Likewise, 2A was reused for administering formulation 2Ac. A switch was performed using the following procedure. The initial bacterial regimen was stopped and a wash out period of 3 to 4 weeks was allowed prior to a switch to a new treatment regimen. Mini pigs in the other groups were subjected exclusively to the assigned food regimen (3A, 3B, 4A, 5A, respectively). All of the screened, chosen and specially employed probiotic microbial strains were Generally Recognized As Safe (GRAS) classification by FDA except those obtained in the formulations of *B. pastuerii.*

Weight measurement and blood draw and analysis were performed at different time intervals. Due to the small sample size, as well as difference in body weights and the measurement intervals, regression analysis and curve fitting were used to assess the data. Generally, these mathematical techniques are used to explain and/or predict additional data points. Present analysis determined changes in body weight, BUN, and creatinine levels for each mini pig. The details of the formula, number of pigs, delivery mode, daily dosage, duration, composition, and a general summary of the findings are listed in Table 3. Compositions 1, 2A, 2B, 2Ac, 3A, 3B and 4A are not according to the invention.

**TABLE 3**

| **Formula (# of pigs)** | **Delivery mode/daily dosage/duration (days)** | **Composition per dose, CFU** | **Summary of Findings** |
|---|---|---|---|
| **1 (6)** | Double layered gelatin capsule/ 1 to 12/27 to 50 | *S. thermophilus, L. bulgaricus, L. acidophilus, B. bifidus* (10x10⁹/cap) (1:1:1:1) | BUN decrease |
| | | | Creatinine slight decrease |
| | | | Weight increase |
| **2A (2)** | Frozen food ball/ 1 to 2/69 | *B. pasteurii* (5x10⁹), *B. coagulans* (10x10⁹) | BUN increase |
| | | | Creatinine slight decrease |
| | | | Weight increase |
| **2B (3)** | Frozen food ball/ 1 to 2/69 | *B. coagulans* (10x10⁹) | BUN slight decrease |
| | | | Creatinine slight decrease |
| | | | Weight stable |
| **2Ac (2)** | Tablet/10/15 | *B. coagulans* (0.78x10⁸/tab) | BUN increase |
| | | | Creatinine slight decrease |
| | | | Weight slight decrease |
| **3A (3)** | Frozen food balls/ 1 to 4/100 | *S. thermophilus* (11.6x10⁹), *L*. *acidophilus* (1x10⁹), *B. longum* (1x10⁹) | BUN stable |
| | | | Creatinine stable |
| | | | Weight stable |
| **3B (2)** | Frozen food balls/ 1 to 4/100 | *L. acidophilus* (1x10⁹), *B. longum* (1x10⁹) | BUN stable |
| | | | Creatinine stable |
| | | | Weight stable |
| **4A (3)** | Frozen food balls/ 1 to 10/51 | *S. thermophilus* (11.6x10⁹), *L. acidophilus* (1x10⁹), *B. longum* (1x10⁹) | BUN stable |
| | | | Creatinine slight decrease |
| | | | Weight increase |
| **5A (2)** | Gelatin capsule/ 3 to 10/20 (ongoing) | *S. thermophilus* (20.4x10⁹), *L. acidophilus* (1x10⁹), *B. longum* (1x10⁹) | BUN decrease |
| | | | Creatinine decrease |
| | | | Weight inconclusive |

Of the several probiotic oral formulations tested, a specific formulation of four microbial strains (Formulation 1) on a low frequency dosage regimen in these 5/6th nephrectomized mini pigs (n=6) exhibited (a) continued gain in body weights-approximately 33%, (b) decreased BUN and as well creatinine levels decreased by approximately 13%. In a similar manner, two mini pigs on formulation 5A also demonstrated decreased BUN and creatinine levels, although the body weight of one mini pig increased and that of the other decreased. In addition, the formulations 3A, 3b and 4a demonstrated stable or slight increases in body weight, somewhat stable or slight decreases in levels of urea and creatinine. In general, all of these data and findings reflect that at least nitrogenous waste metabolites (urea and creatinine) were not accumulating in the blood. These results show that a suitable combination of selectively chosen probiotic bacteria are suitable for oral gut-based uremia therapy.

Formulation 5A forms part of the present invention.

### Comparative Example 2: Health bar Recipe for Preparation under Room and Lower Storage Temperature

### Ingredients:

1 cup oat bran, ½ cup toasted sunflower and/or sesame seeds, or peanut suitably ground in a dry grinder or kitchen mill
½ cup low fat milk or soy milk powder - may be added with maltodextrin or fructose or aspartame to appropriate sweetness
½ cup raisins plus 2 tablespoons carob powder.
Mix well, then add to ½ cup white or brown rice, cooked, dehydrated and pulverized and (using a food processor again), add ½ cup peanut butter (more or less, depending on consistency)
½ cup honey or high fructose corn syrup, about 1 mL of vanilla essence (natural).

### Procedure:

All ingredients are combined and kneaded (if needed, more oat bran or rolled oats can be added) until thoroughly mixed. A cake mixer works well for this. A microbial cocktail powder mix (approximately 10 billion cfu/gm) is then added to the mixture. The ingredients are mixed well again. The mixture can be reasonably soft, and cooling for about 24 hours will help to bind all ingredients together. The bars are rolled or pressed out to about 1 cm thick and cut into suitable size. This recipe makes about 16 bars, with the preferred size of approximately 1 cm x 1.5 cm x 6 cm.

### Comparative Example 3: Health Bar Recipe for Non-Refrigerated Conditions

Health bars are prepared at 35°C to 40°C for a brief period of time and stored desirably at room temperature (non-refrigerated conditions). The dry ingredients are combined and mixed uniformly (as described in Example 2). The liquid ingredients are then added and heated to approximately 50°C to 60°C. The mixture is allowed to cool with continued mixing. The product will become more viscous. When the temperature is between 35°C to 40°C, the microbial cocktail mixture (approximately 10 billion cfu/gm) is added. The entire product is poured in a lined tray or lightly greased with clarified butter. The product is immediately allowed to cool and settle in a refrigerator for 12 to 24 hours until sufficiently hard, but easy to cut, into the required size. The product is cut to desired pieces and dimensions and wrapped and packed individually.

## Claims

1. A composition for use in augmenting kidney function in a subject, wherein the composition comprises living probiotic bacteria in the form of a capsule containing *Lactobacillus acidophilus* in an amount of 1 x 10⁹ cfu, *Bifidobacterium longum* in an amount of 1 x 10⁹ cfu, and *Streptococcus thermophilus* in an amount of 2.04 x 10¹⁰ cfu, wherein said probiotic bacteria reduce creatinine and blood urea nitrogen levels in the subject.

2. A composition for use of claim 1 for augmenting kidney function by reducing the build up of toxins, build up of metabolic wastes, and overgrowth of undesirable bacteria.

3. The composition for use of claim 2, wherein the composition is administered to the subject to alleviate the symptoms of uremia.

4. The composition for use of claim 2, wherein the composition is administered to the subject to alleviate the increased creatinine or blood urea nitrogen levels in subjects undergoing cancer, HIV or AIDS chemotherapy treatment.

5. The composition for use of claim 2, wherein the composition is administered to the subject to alleviate the increased creatinine or blood urea nitrogen levels in subjects with metabolic syndrome.

6. The composition for use of claim 2, wherein the composition is administered to the subject to alleviate the symptoms of increased creatinine or blood urea nitrogen levels in subjects consuming high protein and low carbohydrate diets.

## Patentansprüche

1. Zusammensetzung für die Verwendung bei der Verbesserung der Nierenfunktion bei einem Subjekt, wobei die Zusammensetzung lebende probiotische Bakterien in Form einer Kapsel umfasst, die *Lactobacillus acidophilus* in einer Menge von 1 x 10⁹ KBE, *Bifidobacterium longum* in einer Menge von 1 x 10⁹ KBE und *Streptococcus thermophilus* in einer Menge von 2,04 x 10¹⁰ KBE enthält, wobei die probiotischen Bakterien Kreatinin- und Blut-Harnstoffstickstoff-Spiegel bei dem Subjekt reduzieren.

2. Zusammensetzung für die Verwendung nach Anspruch 1 für die Verbesserung der Nierenfunktion durch Reduzierung der Ansammlung von Toxinen, Ansammlung von Stoffwechselabfällen und des übermäßigen Wachstums von unerwünschten Bakterien.

3. Zusammensetzung für die Verwendung nach Anspruch 2, wobei die Zusammensetzung an das Subjekt verabreicht wird, um die Symptome von Urämie zu lindern.

4. Zusammensetzung für die Verwendung nach Anspruch 2, wobei die Zusammensetzung an das Subjekt verabreicht wird, um die erhöhten Kreatinin- oder Blut-Harnstoffstickstoff-Spiegel bei Subjekten, die sich einer Krebs-, HIV- oder AIDS-Chemotherapiebehandlung unterziehen, zu vermindern.

5. Zusammensetzung für die Verwendung nach Anspruch 2, wobei die Zusammensetzung an das Subjekt verabreicht wird, um die erhöhten Kreatinin- oder Blut-Harnstoffstickstoff-Spiegel bei Subjekten mit metabolischem Syndrom zu vermindern.

6. Zusammensetzung für die Verwendung nach Anspruch 2, wobei die Zusammensetzung an das Subjekt verabreicht wird, um die Symptome von erhöhten Kreatinin- oder Blut-Harnstoffstickstoff-Spiegeln bei Subjekten, die eine proteinreiche und kohlenhydratarme Ernährung konsumieren, zu lindern.

## Revendications

1. Composition pour une utilisation destinée à augmenter la fonction rénale chez un sujet, où la composition comprend des bactéries probiotiques vivantes sous la forme d'une capsule contenant *Lactobacillus acidophilus* selon une quantité de 1 × 10⁹ ufc, *Bifidobacterium longum* selon une quantité de 1 × 10⁹ ufc, et *Streptococcus thermophilus* selon une quantité de 2,04 × 10¹⁰ ufc, où lesdites bactéries probiotiques réduisent les taux de créatinine et d'azote uréique du sang chez le sujet.

2. Composition pour une utilisation selon la revendication 1 destinée à augmenter la fonction rénale par la réduction de l'accumulation de toxines, l'accumulation de déchets métaboliques, et la prolifération de bactéries indésirables.

3. Composition pour une utilisation selon la revendication 2, où la composition est administrée au sujet afin de soulager les symptômes de l'urémie.

4. Composition pour une utilisation selon la revendication 2, où la composition est administrée au sujet afin de soulager les taux accrus de créatinine ou d'azote uréique du sang chez des sujets sous traitement de chimiothérapie contre le cancer, le VIH ou le SIDA.

5. Composition pour une utilisation selon la revendication 2, où la composition est administrée au sujet afin de soulager les taux accrus de créatinine ou d'azote uréique du sang chez des sujets souffrant d'un syndrome métabolique.

6. Composition pour une utilisation selon la revendication 2, où la composition est administrée au sujet afin de soulager les symptômes de taux accrus de créatinine ou d'azote uréique du sang chez des sujets consommant des régimes alimentaires riches en protéines et faibles en glucides.
